# EUROPÄISCHE PATENTANMELDUNG

(11) **EP 3 309 574 A1**
(43) Veröffentlichungstag der Anmeldung: **18.04.2018**
(21) Anmeldenummer: 17172144.2
(22) Anmeldetag: 22.05.2017
(51) Int. Cl.: G01R 33/54, G01R 33/483, G01R 33/565

(54) **VERFAHREN ZUM BETRIEB EINER MAGNETRESONANZANLAGE, DATENTRÄGER SOWIE MAGNETRESONANZANLAGE**

(71) Anmelder: Siemens Healthcare GmbH, 91052 Erlangen (DE)
(72) Erfinder: Paul, Dominik, 91088 Bubenreuth (DE); Schmitt, Peter, 91085 Weisendorf (DE)

(57) **Zusammenfassung**

Die Erfindung betrifft ein zum Betrieb einer Magnetresonanzanlage mit den Schritten: Bereitstellen eines Magnetresonanzbilddatensatzes, wobei wenigstens ein Bild des Magnetresonanzbilddatensatzes verzerrt ist, Bereitstellen eines Selektionssymbols zur Selektion eines Messvolumens, wobei das Selektionssymbol so verzerrt ist, dass es nach einer Verzerrungskorrektur einen rechteckigen Querschnitt aufweist, und Überlagerung des Selektionssymbols auf ein Bild des Magnetresonanzbilddatensatzes.

Die Erfindung betrifft ferner einen Datenträger.

Die Erfindung betrifft ferner eine Magnetresonanzanlage, mit der das vorgenannte Verfahren ausführbar ist.

## Beschreibung

Bei Magnetresonanzuntersuchungen werden vor Beginn der eigentlichen Experimente zum Gewinnen von anatomischen oder funktionellen Informationen, sogenannte Justagemessungen, vorgenommen.

Beispielsweise wird das Magnetfeld homogenisiert und die Resonanzfrequenz ermittelt. Hierfür existieren automatisierte Routinen.

Weiterhin sind die zu messenden Schichten oder Volumina festzulegen. Die hierfür verwendeten Übersichtsaufnahmen werden auch Scoutscans genannt. Diese Messvolumenfestlegung geschieht üblicherweise von Hand, da die Platzierung und Zahl der Messschichten einerseits von einer Vielzahl an Randbedingungen abhängt und andererseits keine Automatisierung mit ausreichender Genauigkeit für alle Fragestellungen vorhanden ist.

Je nach Messparametern und verwendeter Sequenz kann es dabei zu Verzerrungen in den Bilddaten der Justagemessungen kommen. Insbesondere schnelle Messsequenzen wie TrueFisp und EPI sind anfällig für eine ganze Reihe an Artefakten. Verzerrungen entstehen auch, wenn die Scoutscans Volumenbereiche abdecken, die aus dem Isozentrum der Magnetresonanzanlage herausreichen. Als Isozentrum wird der homogene B0-Bereich in der Mitte des Magnetfeldes bezeichnet.

Verzerrungen, die bei MR-Verfahren auftreten und sich negativ auswirken, sind in vielfältigen Zusammenhängen bekannt. Lediglich beispielhaft seien die DE 102014210778 B4, die DE 102014214844 B4, die DE 102014219291 A1 und die DE 102015204483 A1 genannt.

Die Verzerrungen werden dabei üblicherweise durch die Steuerungseinrichtung automatisch korrigiert. Dadurch können die Justagemessungen oder andere verzerrt aufgenommene Bilder nicht direkt als Scoutscans verwendet werden, um die Positionierungsgenauigkeit des Messvolumens zu wahren. Die verzerrungskorrigierten Bilddaten müssen redundant prozessiert und ohne Verzerrungskorrektur dargestellt werden, um sie für Positionierungsschritte zu verwenden.

Es ist daher Aufgabe der vorliegenden Erfindung, ein Verfahren anzugeben, bei dem eine optimale Positionierungsgenauigkeit bei möglichst geringem Rechenaufwand ermöglicht wird.

Diese Aufgabe wird gelöst durch ein Verfahren zum Betrieb einer Magnetresonanzanlage mit den Schritten:
- Bereitstellen eines Magnetresonanzbilddatensatzes, wobei wenigstens ein Bild des Magnetresonanzbilddatensatzes verzerrungskorrigiert ist,
- Bereitstellen wenigstens eines Selektionssymbols zur Selektion eines Messvolumens, wobei das Selektionssymbol zumindest teilweise verzerrt ist, und
- Überlagerung des Selektionssymbols auf ein verzerrungskorrigiertes Bild des Magnetresonanzbilddatensatzes.

Als Kern der Erfindung wird dabei angesehen, nicht die Verzerrung der Bilddaten wiederherzustellen sondern das Selektionssymbol entsprechend der korrigierten Verzerrung im Bild zu verzerren. Auf diese Art und Weise kann die Positionierungsgenauigkeit erhalten bleiben, da das dargestellte und das zu messende Volumen wieder übereinstimmen.

Die Schritte zur Durchführung des Verfahrens werden vorzugsweise vollständig automatisiert von einer Steuerungseinrichtung der Magnetresonanzanlage ausgeführt.

Durch die Verzerrung des Selektionssymbols kann also die wahre Form des Untersuchungsobjektes bei der Messung, die im verzerrungskorrigierten Bild nicht mehr sichtbar ist, dargestellt werden.

Dadurch wird eine mehrmalige Prozessierung der Bilder vermieden. Auch werden so Unklarheiten vermieden. Sieht der Benutzer unverzerrte Bilder, so kann dies daran liegen, dass die Bilddaten keinerlei Verzerrungen aufweisen, da das Bild den Untersuchungsbereich um das Isozentrum herum abbildet. Alternativ können die Bilder auch verzerrungskorrigiert sein. Der Benutzer muss also nicht mehr nachforschen, ob er zur Selektion eines Messvolumens erst eine Verzerrungskorrektur rückgängig machen muss.

Ob das Bild, mit dem die Positionierung vorgenommen wird, in Bereichen oder überall verzerrt ist ergibt sich aus der Form des Selektionssymbols. Die Verzerrung des Selektionssymbols kann ohne nennenswerten Rechenaufwand und spezifisch bei der Positionierung des Untersuchungsbereichs durch die Steuerungseinrichtung durchgeführt werden.

Dabei kann je nach zugrunde liegendem Magnetresonanzbilddatensatz ein einziges zweidimensionales Bild vorhanden sein oder auch mehrere. Ein dreidimensionaler Bilddatensatz wird dabei auch als aus mehreren zweidimensionalen Bildern bestehend betrachtet. Ein Selektionssymbol wird aber üblicherweise auf einem zweidimensionalen Bild positioniert. Dazu werden entweder bereits zweidimensional aufgenommene Bilder verwendet oder entsprechende Bilder aus einem dreidimensionalen Bilddatensatz berechnet. Bilder sind dabei die Darstellung des Bilddatensatzes.

Der Unterschied zwischen einem Bild und einem Bilddatensatz ist in der folgenden Erfindung folgendermaßen: Ein Bilddatensatz enthält die Messdaten, die zur Rekonstruktion eines oder mehrerer Bilder maßgeblich sind. Bei paralleler Bildgebung wird z.B. noch auf Kalibrierungsdaten aus einem anderen Datensatz zurückgegriffen. Ausgehend von diesen Daten können unterschiedliche Post-Prozessierungsschritte vorgenommen werden, um aus dem Bilddatensatz ein Bild zu generieren. Bspw. kann ein 128 x 128 Messpunkte aufweisender Bilddatensatz mit unterschiedlichen Zerofilling-Faktoren auf Bilder der Größe 128 x 128, 256 x 256 oder auch 512 x 512 prozessiert werden.

Der Bilddatensatz ist immer der gleiche, die Bilder unterscheiden sich aber. Die Bilder sind auch das, was der Benutzer sieht.

Abkürzend wird statt Magnetresonanzbilddatensatz auch nur Bilddatensatz verwendet.

Vorzugsweise kann die Verzerrung des Selektionssymbols ortsabhängig vorgenommen werden. Die Verzerrung des Bildes kann ortsabhängig sein, z.B. in der Mitte schwächer als an den Rändern. Durch eine möglichst genaue Nachbildung der Verzerrung des Bildes bzw. des Untersuchungsbereiches kann die Positionierungsgenauigkeit des Selektionssymbols optimiert werden.

Vorzugsweise ist das Selektionssymbol so verzerrt, dass es nach einer Verzerrungskorrektur einen rechteckigen Querschnitt aufweist. Bevorzugt kann der Querschnitt quadratisch sein. Die Vorschrift zur Verzerrungskorrektur des Bildes führt also auch zu einer Verzerrungskorrektur des Selektionssymbols.

Vorzugsweise kann die Verzerrung des Selektionssymbols in Abhängigkeit wenigstens eines physiologischen Parameters vorgenommen werden. Physiologische Parameter sind beispielsweise Fluss und Bewegung. Während Fließrichtungen und - geschwindigkeiten aus Erfahrungswerten ableitbar sind können Bewegungen mit Navigatoren ermittelt werden.

Vorteilhafterweise kann die Verzerrung des Selektionssymbols in Abhängigkeit wenigstens eines gradientenbasierten Parameters, insbesondere der Stärke wenigstens eines Gradientenfeldes, vorgenommen werden. Abweichungen zwischen dem Soll-Gradienten und dem Ist-Gradienten führen zu Fehlkodierungen der Ortsposition und damit zu Verzerrungen. Sind die Abweichungen bekannt oder abschätzbar ist auch eine entsprechende Verzerrung des Selektionssymbols möglich.

Vorteilhafterweise kann die Verzerrung des Selektionssymbols in Abhängigkeit der Tischposition des Patiententisches vorgenommen werden. Die Tischposition kann als Maß für gradientenbasierte Verzerrungen benutzt werden. Die Verzerrung ist damit auch ortsabhängig.

Vorzugsweise kann das Selektionssymbol zeilenweise verzerrt sein. Es kann also bspw. in Abhängigkeit der Tischposition für jede k-Raum-Zeile eine eigene Verzerrungsvorschrift vorhanden sein. Die Verzerrungsvorschrift kann weiterhin in Abhängigkeit der Auflösung des Magnetresonanzdatensatzes variieren. Für jede Raumrichtung kann dabei eine eigene Vorschrift existieren.

Vorzugsweise kann die Verzerrung des Selektionssymbols in Abhängigkeit wenigstens eines physikalischen Parameters des Untersuchungsobjektes vorgenommen werden. Beispielsweise führt der sogenannte chemical shift zu einer Verschiebung von Fettsignal gegenüber Wassersignal. Suszeptibilitätssprünge im Untersuchungsobjekt wirken als Gradienten und führen so ebenfalls zu Fehlkodierungen der Ortsinformation und auch zu Signalauslöschungen.

Vorzugsweise kann die Verzerrung des Selektionssymbols in Abhängigkeit wenigstens eines messsequenzabhängigen Parameters vorgenommen werden. Derartige Verzerrungen, die aus kalkulierbaren Verzerrungen aufgrund des Aufbaus der Messsequenz resultieren, lassen sich mathematisch erfassen und dadurch besonders exakt nachbilden.

Je nach Ausgestaltung des Magnetresonanzbilddatensatzes sind Verzerrungen des Selektionssymbols in zwei oder drei Raumrichtungen möglich.

Vorteilhafterweise kann die Verzerrung in Phasenrichtung vorgenommen werden. Zusätzlich oder alternativ kann die Verzerrung in Leserichtung vorgenommen werden. Zusätzlich oder alternativ kann die Verzerrung in Schichtauswahlrichtung vorgenommen werden.

Dargestellt und einem Bild überlagert werden können immer nur Verzerrungen in zwei Raumrichtungen. Allerdings können mehrere einzelne Bilder oder Schnitte bzw. Bilder eines dreidimensionalen Bilddatensatzes verwendet werden, um ein Messvolumen festzulegen. Dabei können insgesamt auch alle drei Raumrichtungen bzw. Gradientenrichtungen berücksichtigt werden.

Selbstverständlich können auch Bilder verwendet werden, die nicht genau in Lese- und Phasenrichtung oder Schichtauswahlrichtung positioniert sind. Dann wird die Verzerrung durch eine Projektion der Phasen-, Lese- und Schichtauswahlrichtung auf die Richtung des Bildes erhalten.

Vorzugsweise kann die Verzerrung des Selektionssymbols durch eine Inversion der Verzerrungskorrektur des Bilddatensatzes erhalten werden. Wie bereits beschrieben wird die Positionierungsgenauigkeit umso mehr erhöht je besser die Verzerrung beschrieben werden kann. Ist eine Inversion der Verzerrungskorrektur möglich führt dies zu einer optimalen Verzerrung des Selektionssymbols.

Vorzugsweise kann als Selektionssymbol ein Gitter verwendet werden. Alternativ kann als Selektionssymbol ein Viereck verwendet werden. Übliche Selektionssymbole sind quadratisch oder zumindest rechteckig. Diese werden durch eine Verzerrung zu unregelmäßigeren Vierecken, bspw. einem Parallelogramm, umgewandelt.

Durch ein Selektionssymbol wird unabhängig von der Messsequenz immer ein Messvolumen ausgewählt, da auch bei Messungen mit einer einzigen Schicht diese eine bestimmte Dicke hat und so ein Messvolumen definiert.

Vorteilhafterweise kann das Selektionssymbol zur Selektion eines Messvolumens für eine spektroskopische Messung verwendet werden. Mittels eines Vierecks kann z.B. ein Messvolumen für eine reine Spektroskopiemessung und mittels eines Gitters ein Messvolumen für ein sogenanntes chemical shift image (CSI) festgelegt werden.

Daneben betrifft die Erfindung einen Datenträger für eine Steuerungseinrichtung zur Steuerung einer Datenerzeugungseinheit einer Magnetresonanzanlage mit Daten zum Durchführen des beschriebenen Verfahrens. Vorteilhafterweise kann die Datenerzeugungseinheit eine Bilderzeugungseinheit sein.

Daneben betrifft die Erfindung eine Magnetresonanzanlage mit einer Steuerungseinrichtung. Die Magnetresonanzanlage zeichnet sich dadurch aus, dass die Steuerungseinrichtung zur Durchführung des Verfahrens wie beschrieben ausgebildet ist.

Die Implementierung der vorgenannten Verfahren in der Steuervorrichtung kann dabei als Software oder aber auch als (fest verdrahtete) Hardware erfolgen.

Weitere vorteilhafte Ausgestaltungen der erfindungsgemäßen Magnetresonanzanlage korrespondieren zu entsprechenden Ausgestaltungen des erfindungsgemäßen Verfahrens. Zur Vermeidung unnötiger Wiederholungen wird somit auf die entsprechenden Verfahrensmerkmale und deren Vorteile verwiesen.

Weitere Vorteile, Merkmale und Besonderheiten der vorliegenden Erfindung ergeben sich aus der nachfolgenden Beschreibung vorteilhafter Ausgestaltungen der Erfindung.

Dabei zeigen:
- Fig. 1: eine Magnetresonanzanlage,
- Fig. 2: ein Planungsbild mit einem Selektionssymbol (Stand der Technik), und
- Fig. 3: ein Planungsbild mit einem Selektionssymbol.

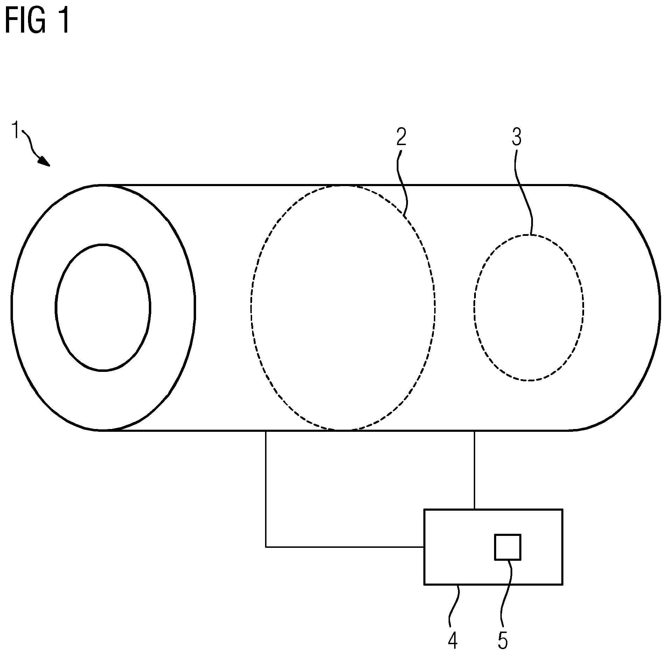

Figur 1 zeigt eine Magnetresonanzanlage 1. Diese weist eine Sendespulenanordnung 2 und eine Empfangsspulenanordnung 3 auf. Die Empfangsspulenanordnung 3 kann als Spulenarray ausgebildet sein.

Zur Steuerung der Magnetresonanzanlage 1 ist eine Steuerungseinrichtung 4 vorhanden.

Die Magnetresonanzanlage 1 besitzt weiterhin einen Datenträger 5. Der Datenträger 5 kann als Teil der Steuerungseinrichtung 4 ausgebildet sein oder unabhängig davon. Auf dem Datenträger 5 sind Computerprogramme zur Durchführung von Magnetresonanzmessungen abgespeichert.

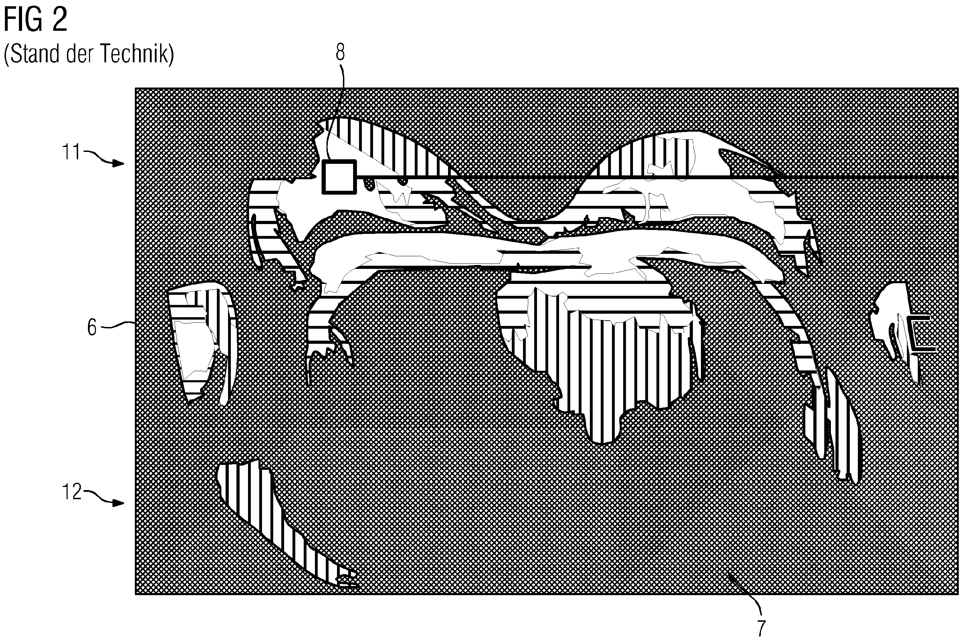

Figur 2 zeigt ein bekanntes Planungsbild 6. Das Planungsbild 6 bildet den Untersuchungsbereich 7, einen Querschnitt auf Höhe des Brustkorbs, aufgrund von Gradientenfehlern mit Verzerrungen ab. Dementsprechend ist der Untersuchungsbereich 7 in einem ersten Abschnitt 11 stärker verzerrt als in einem zweiten Abschnitt 12. Die Verzerrungen sind insbesondere von der Tischposition des Patententisches abhängig.

Die Verzerrung ist exemplarisch links nach rechts dargestellt. Diese kann in Lese-, Phasen-, Schichtauswahlrichtung oder einer Kombination daraus liegen. Die Verzerrungen können in allen Richtungen vorkommen.

Dem Planungsbild 6 ist ein Selektionssymbol 8 überlagert, mit dem das Messvolumen einer spektroskopischen Messung festgelegt werden kann. Das Selektionssymbol 8 ist quadratisch. Wird das Messvolumen in mehreren Ansichten, insbesondere in aufeinander senkrecht stehenden Bildern, festgelegt, kann es in einer anderen Ansicht auch rechteckig ausgestaltet sein. Das Messvolumen ist dann quaderförmig oder würfelförmig.

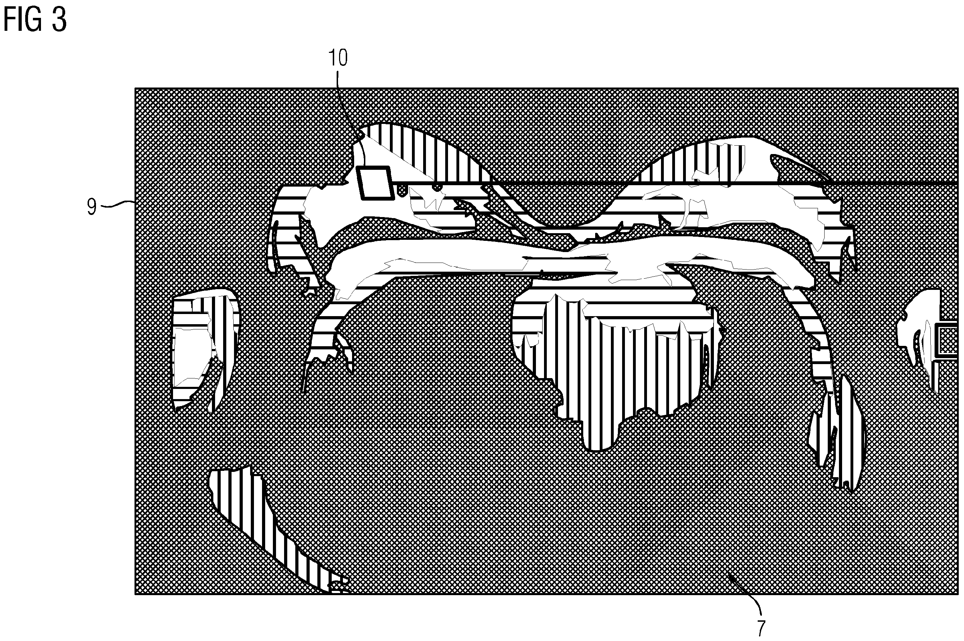

Figur 3 zeigt ein Planungsbild 9. Das Planungsbild 9 ist mit den gleichen Gradientenfehlern wie das Planungsbild 6 aufgenommen, allerdings wurde es verzerrungskorrigiert. Es sieht daher aus wie ein Bild, das mit exakt konstanten Gradienten aufgenommen wurde.

Das Selektionssymbol 10 ist entsprechend der Verzerrung des Gradientenfeldes verzerrt. Eine Verzerrungskorrektur des Selektionssymbols 10 überführt dieses in ein Quadrat, wie zum Selektionssymbol 8 in Figur 2 gezeigt. Dieselbe Verzerrungskorrektur überführt den verzerrten Untersuchungsbereich 7 in Figur 2 auch in einen verzerrungskorrigierten Untersuchungsbereich 7 und damit in ein verzerrungskorrigiertes Planungsbild 9 wie in Figur 3.

Durch die Verwendung eines verzerrten Selektionssymbols 10 wird es möglich, auch verzerrungskorrigierte Bilder als Planungsbilder 9 zu verwenden, ohne dass die Positionierungsgenauigkeit des Messvolumens leidet.

## Patentansprüche

1. Verfahren zum Betrieb einer Magnetresonanzanlage (1) mit den Schritten:
- Bereitstellen eines Magnetresonanzbilddatensatzes, wobei wenigstens ein Bild (9) des Magnetresonanzbilddatensatzes verzerrungskorrigiert ist,
- Bereitstellen eines Selektionssymbols (10) zur Selektion eines Messvolumens, wobei das Selektionssymbol (10) zumindest teilweise verzerrt ist, und
- Überlagerung des Selektionssymbols (10) auf ein verzerrungskorrigiertes Bild (9) des Magnetresonanzbilddatensatzes.

2. Verfahren nach Anspruch 1, **dadurch gekennzeichnet, dass** die Verzerrung des Selektionssymbols (10) ortsabhängig vorgenommen wird.

3. Verfahren nach Anspruch 1 oder 2, **dadurch gekennzeichnet, dass** die Verzerrung des Selektionssymbols (10) in Abhängigkeit wenigstens eines physiologischen Parameters vorgenommen wird.

4. Verfahren nach einem der vorangehenden Ansprüche, **dadurch gekennzeichnet, dass** die Verzerrung des Selektionssymbols (10) in Abhängigkeit wenigstens eines gradientenbasierten Parameters vorgenommen wird.

5. Verfahren nach einem der vorangehenden Ansprüche, **dadurch gekennzeichnet, dass** die Verzerrung des Selektionssymbols (10) in Abhängigkeit wenigstens eines physikalischen Parameters des Untersuchungsobjektes vorgenommen wird.

6. Verfahren nach einem der vorangehenden Ansprüche, **dadurch gekennzeichnet, dass** die Verzerrung des Selektionssymbols (10) in Abhängigkeit wenigstens eines messsequenzabhängigen Parameters vorgenommen wird.

7. Verfahren nach einem der vorhergehenden Ansprüche, **dadurch gekennzeichnet, dass** die Verzerrung in Phasenrichtung vorgenommen wird.

8. Verfahren nach einem der vorhergehenden Ansprüche, **dadurch gekennzeichnet, dass** die Verzerrung in Leserichtung vorgenommen wird.

9. Verfahren nach einem der vorhergehenden Ansprüche, **dadurch gekennzeichnet, dass** die Verzerrung in Schichtauswahlrichtung vorgenommen wird.

10. Verfahren nach einem der vorhergehenden Ansprüche, **dadurch gekennzeichnet, dass** die Verzerrung des Selektionssymbols (10) durch eine Inversion der Verzerrungskorrektur des Magnetresonanzbilddatensatzes erhalten wird.

11. Verfahren nach einem der vorhergehenden Ansprüche, **dadurch gekennzeichnet, dass** als Selektionssymbol (10) ein Gitter verwendet wird.

12. Verfahren nach einem der Ansprüche 1 bis 10, **dadurch gekennzeichnet, dass** als Selektionssymbol (10) ein Viereck verwendet wird.

13. Verfahren nach einem der vorhergehenden Ansprüche, **dadurch gekennzeichnet, dass** das Selektionssymbol (10) zur Selektion eines Messvolumens für eine spektroskopische Messungen verwendet wird.

14. Datenträger (5) für eine Steuerungseinrichtung (4) zur Steuerung einer Datenerzeugungseinheit, insbesondere Bilderzeugungseinheit, einer Magnetresonanzanlage (1) mit Daten zum Durchführen eines Verfahrens gemäß einem der vorhergehenden Ansprüche.

15. Magnetresonanzanlage (1) mit einer Steuerungseinrichtung (4), **dadurch gekennzeichnet, dass** die Steuerungseinrichtung (4) zur Durchführung eines Verfahrens nach einem der Ansprüche 1 bis 13 ausgebildet ist.

## Geänderte Patentansprüche

### Geänderte Patentansprüche gemäss Regel 137(2) EPÜ.

1. Verfahren zum Betrieb einer Magnetresonanzanlage (1) mit den Schritten:
- Bereitstellen eines Magnetresonanzbilddatensatzes, wobei wenigstens ein Bild (9) des Magnetresonanzbilddatensatzes verzerrungskorrigiert ist,
- Bereitstellen eines Selektionssymbols (10) zur Selektion eines Messvolumens, wobei das Selektionssymbol (10) zumindest teilweise verzerrt ist,
- Überlagerung des Selektionssymbols (10) auf ein verzerrungskorrigiertes Bild (9) des Magnetresonanzbilddatensatzes,
- wobei die Verzerrung des Selektionssymbols (10) durch eine Inversion der Verzerrungskorrektur des Magnetresonanzbilddatensatzes erhalten wird, und
- die Verzerrung in Phasenrichtung und/oder in Leserichtung vorgenommen wird.

2. Verfahren nach Anspruch 1, **dadurch gekennzeichnet, dass** die Verzerrung des Selektionssymbols (10) ortsabhängig vorgenommen wird.

3. Verfahren nach Anspruch 1 oder 2, **dadurch gekennzeichnet, dass** die Verzerrung des Selektionssymbols (10) in Abhängigkeit wenigstens eines physiologischen Parameters vorgenommen wird.

4. Verfahren nach einem der vorangehenden Ansprüche, **dadurch gekennzeichnet, dass** die Verzerrung des Selektionssymbols (10) in Abhängigkeit wenigstens eines gradientenbasierten Parameters vorgenommen wird.

5. Verfahren nach einem der vorangehenden Ansprüche, **dadurch gekennzeichnet, dass** die Verzerrung des Selektionssymbols (10) in Abhängigkeit wenigstens eines physikalischen Parameters des Untersuchungsobjektes vorgenommen wird.

6. Verfahren nach einem der vorangehenden Ansprüche, **dadurch gekennzeichnet, dass** die Verzerrung des Selektionssymbols (10) in Abhängigkeit wenigstens eines messsequenzabhängigen Parameters vorgenommen wird.

7. Verfahren nach einem der vorhergehenden Ansprüche, **dadurch gekennzeichnet, dass** die Verzerrung in Schichtauswahlrichtung vorgenommen wird.

8. Verfahren nach einem der vorhergehenden Ansprüche, **dadurch gekennzeichnet, dass** als Selektionssymbol (10) ein Gitter verwendet wird.

9. Verfahren nach einem der Ansprüche 1 bis 7, **dadurch gekennzeichnet, dass** als Selektionssymbol (10) ein Viereck verwendet wird.

10. Verfahren nach einem der vorhergehenden Ansprüche, **dadurch gekennzeichnet, dass** das Selektionssymbol (10) zur Selektion eines Messvolumens für eine spektroskopische Messungen verwendet wird.

11. Datenträger (5) für eine Steuerungseinrichtung (4) zur Steuerung einer Datenerzeugungseinheit, insbesondere Bilderzeugungseinheit, einer Magnetresonanzanlage (1) mit Daten zum Durchführen eines Verfahrens gemäß einem der vorhergehenden Ansprüche.

12. Magnetresonanzanlage (1) mit einer Steuerungseinrichtung (4), **dadurch gekennzeichnet, dass** die Steuerungseinrichtung (4) zur Durchführung eines Verfahrens nach einem der Ansprüche 1 bis 10 ausgebildet ist.
